(19) Europäisches Patentamt
European Patent Office
Office européen des brevets

(11) Veröffentlichungsnummer: **0 036 839**
**A2**

(12) # EUROPÄISCHE PATENTANMELDUNG

(21) Anmeldenummer: **81810098.4**

(51) Int. Cl.³: **A 01 N 47/18**, C 07 D 239/56

(22) Anmeldetag: **16.03.81**

(30) Priorität: **20.03.80 CH 2197/80**
**13.02.81 CH 972/81**

(43) Veröffentlichungstag der Anmeldung: **30.09.81**
**Patentblatt 81/39**

(84) Benannte Vertragsstaaten: **AT BE CH DE FR GB IT LI NL**

(71) Anmelder: **CIBA-GEIGY AG, Patentabteilung Postfach, CH-4002 Basel (CH)**

(72) Erfinder: **Gsell, Laurenz, Dr., Maiengasse 56, CH-4056 Basel (CH)**
Erfinder: **Meyer, Willy, Talstrasse 49, CH-4125 Riehen (CH)**

(54) **4-N,N-Dimethylcarbamoyloxy-5-methyl-pyrimidine, Verfahren zu ihrer Herstellung und ihre Verwendung in der Schädlingsbekämpfung, sowie die als Zwischenprodukte verwendeten 4-Hydroxy-5-methyl-pyrimidine.**

(57) 4-N,N-Dimethylcarbamoyloxy-5-methyl-pyrimidine der Formel

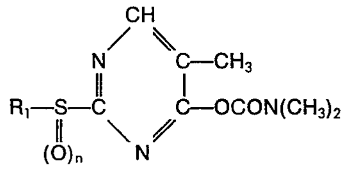

worin $R_1$ $C_1$-$C_4$-Alkyl, $C_1$-$C_4$-Alkylthio-$C_1$-$C_4$-alkyl, $C_1$-$C_4$-Alkoxy-$C_1$-$C_4$-alkyl, $C_1$-$C_4$-Halogenalkyl, $C_1$-$C_4$-Cyanoalkyl, $C_2$-$C_5$-Alkenyl, $C_2$-$C_5$-Halogenalkenyl oder $C_2$-$C_5$-Alkinyl und n die Zahlen 0, 1 oder 2 bedeutet. Ein Verfahren zu deren Herstellung und ihre Verwendung in der Schädlingsbekämpfung sowie deren Zwischenprodukte werden beschrieben.

EP 0 036 839 A2

CIBA-GEIGY AG
Basel (Schweiz)

5-12771/1+2/=

4-N,N-Dimethylcarbamoyloxy-5-methyl-pyrimidine, Verfahren zu ihrer Herstellung und ihre Verwendung in der Schädlingsbekämpfung, sowie die als Zwischenprodukte verwendeten 4-Hydroxy-5-methyl-pyrimidine.

Die vorliegende Erfindung betrifft 4-N,N-Dimethylcarbamoyloxy-5-methyl-pyrimidine, Verfahren zu ihrer Herstellung und ihre Verwendung in der Schädlingsbekämpfung, sowie die als Zwischenprodukte verwendeten 4-Hydroxy-5-methyl-pyrimidine.

Die Pyrimidine haben die Formel

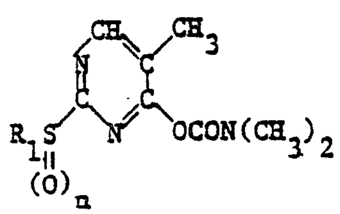

(I),

worin $R_1$ $C_1$-$C_4$-Alkyl, $C_1$-$C_4$-Alkylthio-$C_1$-$C_4$-alkyl, $C_1$-$C_4$-Alkoxy-$C_1$-$C_4$-alkyl, $C_1$-$C_4$-Halogenalkyl, $C_1$-$C_4$-Cyanoalkyl, $C_2$-$C_5$-Alkenyl, $C_2$-$C_5$-Halogenalkenyl oder $C_2$-$C_5$-Alkinyl und n die Zahlen 0, 1 oder 2 bedeuten. Unter Halogen ist Fluor, Chlor, Brom oder Jod zu verstehen.

Beispiele für die bei $R_1$ in Frage kommenden Alkyl-, Alkylthioalkyl-, Alkoxyalkyl-, Halogenalkyl-, Cyanoalkyl-, Alkenyl-, Halogenalkenyl- und Alkinylgruppen sind u.a.: Methyl, Aethyl, Propyl, Isopropyl, n-, i-, sek.-, tert.-Butyl, Methylthiomethyl, Methoxymethyl, Chloräthyl, 3,3,3-Trichlorpropyl, Cyanomethyl, Cyanoäthyl, Allyl, 3,3-Dimethyl-2-allyl, 3,3-Dichlor-2-allyl, Propargyl.

Wegen ihrer Wirkung bevorzugt sind Verbindungen der Formel I, worin $R_1$ $C_1$-$C_4$-Alkyl, $C_1$-$C_3$-Halogenalkyl, $C_1$-$C_3$-Cyanoalkyl, Methylthiomethyl, 3,3-Dimethyl-2-allyl, 3,3-Dichlor-2-allyl oder Propargyl und n die Zahlen 0, 1 oder 2 bedeuten.

Insbesondere bevorzugt sind aber Verbindungen der Formel I, worin $R_1$ Methyl, Aethyl, Propyl, Isopropyl, i-Butyl, 3,3,3-Trichlorpropyl, Cyanomethyl, Cyanoäthyl, Methylthiomethyl, 3,3-Dimethyl-2-allyl, 3,3-Dichlor-2-allyl oder Propargyl und n die Zahl 0 bedeuten.

Die Verbindungen der Formel I werden nach an sich bekannten Methoden z.B. wie folgt hergestellt:

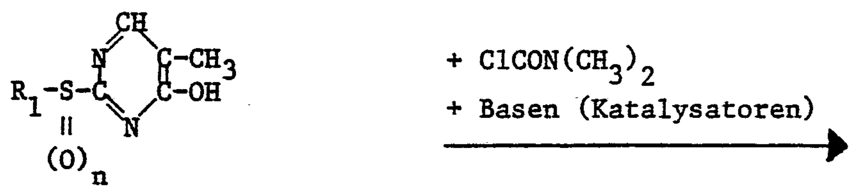

I

II

In der Formel II haben $R_1$ und n die für die Formel I angegebene Bedeutung. Als geeignete Basen (Katalysatoren) kommen insbesondere tertiäre Amine wie Trialkylamine, Dialkylaniline, Pyridine wie z.B. p-Dialkylaminopyridine in Betracht.

Das Verfahren wird bei normalem Druck bei einer Temperatur von -25 bis 150°C, vorzugsweise bei 50 bis 100°C und in einem Lösungs- oder Verdünnungsmittel durchgeführt.

Als Lösungs- oder Verdünnungsmittel eignen sich z.B. Aether und ätherartige Verbindungen wie Diäthyläther, Diisopropyläther, Dioxan und Tetrahydrofuran; aromatische Kohlenwasserstoffe wie Benzol, Toluol und Xylole; Ketone wie Aceton, Methyläthylketon und Cyclohexanon; Nitrile wie Acetonitril; Ester wie Aethylacetat und Butylacetat; sowie Dimethylformamid, Dimethylsulfoxid, Methylcyanid und

halogenierte Kohlenwasserstoffe. Die Ausgangsstoffe der Formel II sind neu; sie können nach bekannten Verfahren hergestellt werden (siehe Beispiel 1).

Die Verbindungen der Formel I eignen sich zur Bekämpfung von Schädlingen an Tieren und Pflanzen. Ferner haben diese Verbindungen fungizide und pflanzenregulatorische Eigenschaften.

Insbesondere eignen sich die Verbindungen der Formel I zur Bekämpfung von Vertretern des Stammes Arthropoda wie der Klasse Insekta, z.B. der Ordnung Lepidoptera, Coleoptera, Homoptera, Heteroptera, Diptera, Thysanoptera Orthoptera Anoplura, Siphonaptera, Mallophaga, Thysanura, Isoptera, Psocoptera und Hymenoptera sowie der Klasse Arachnoida z.B. der Ordnung Acarina.

So eignen sich Verbindungen der Formel I zur Bekämpfung von pflanzenschädigenden Insekten, insbesondere pflanzenschädigenden saugenden und fressenden Insekten, in Zier- und Nutzpflanzen, insbesondere in Baumwollkulturen und Gemüsekulturen.

In diesem Zusammenhang ist hervorzuheben, dass die genannten Verbindungen sich sowohl durch eine stark ausgeprägte systemische als auch Kontakt-Wirkung gegen saugende Insekten insbesondere gegen saugende Insekten der Ordnung Homoptera und vor allem gegen Insekten der Familie Aphididae (wie z.B. Aphis fabae, Aphis craccivora und Myzus persicae), welche sich mit bekannten Mitteln nur schwierig bekämpfen lassen, auszeichnen.

Wirkstoffe der Formel I zeigen auch eine günstige Wirkung gegen Fliegen wie z.B. Musca domestica und Mückenlarven.

Die Verbindungen der Formel I werden in unveränderter Form oder vorzugsweise zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder -äthyl äther, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen wie wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie z.B. die Na- oder K-Salze der Oel- und Stearinsäure, oder von natürlichen Fettsäuregemischen, die z.B. aus Kokosnuss- oder Talgöl gewonnen werden können. Ferner sind auch die Fettsäure-methyl-taurinsalze zu erwähnen.

Häufiger werden jedoch sog. synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxyd-Addukten. Die sulfonierten Benzimidazolderivate enthalten vorzugsweise 2 Sulfonsäuregruppen und einen Fettsäurerest mit 8 - 22 C-Atomen. Alkylarylsulfonate sind z.B. die Na-, Ca- oder Triäthanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxyd-Adduktes in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglycoläther, Polypropylen-Polyäthylenoxydaddukte, Tributylphenoxypolyäthoxyäthanol, Polyäthylenglykol und Octylphenoxypolyäthoxyäthanol erwähnt.

0036839

Ferner kommen auch Fettsäureester von Polyoxyäthylensorbitan wie das Polyoxyäthylensorbitan-trioleat in Betracht.

Bei den kationischen Tensiden handelt es sich vor allem um quartäre Ammoniumsalze, welche als N-Substitutenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyldi(2-chloräthyl)äthylammoniumbromid.

Die in der Formulierungstechnik gebräuchlichen Tenside sind u.a. in folgenden Publikationen beschrieben:

"Mc Cutcheon's Detergents and Emulsifiers Annual" MC Publishing Corp., Ringwood New Jersey, 1979

Die pestiziden Zubereitungen enthalten in der Regel 0,1 bis 99%, insbesondere 0,1 bis 95%, Wirkstoff der Formel I, 1 bis 99,9% eines festen oder flüssigen Zusatzstoffes und 0 bis 25%, insbesondere 0,1 bis 25%, eines Tensides.

Während als Handelsware eher konzentrierte Mittel bevorzugt werden, verwendet der Endverbraucher in der Regel verdünnte Mittel.

Die Mittel können auch weitere Zusätze wie Stabilitsatoren, Entschäumer, Viskositätsregulatoren, Bindemittel, Haftmittel sowie Dünger oder andere Wirkstoffe zur Erzielung spezieller Effekte enthalten.

Formulierungsbeispiele für flüssige Wirkstoffe der Formel I

(% = Gewichtsprozent)

| 1. Emulsions-Konzentrate | a) | b) | c) |
|---|---|---|---|
| Wirkstoff | 20% | 40% | 50% |
| Ca-Dodecylbenzolsulfonat | 5% | 8% | 5,8% |
| Ricinusöl-polyäthylenglykoläther (36 Mol AeO) | 5% | - | - |
| Tributylphenoyl-polyäthylenglykoläther (30 Mol AeO) | - | 12% | 4,2% |
| Cyclohexanon | - | 15% | 20% |
| Xylolgemisch | 70% | 25% | 20% |

Aus solchen Konzentraten können durch Verdünnen mit Wasser Emulsionen jeder gewünschten Konzentration hergestellt werden.

| 2. Lösungen | a) | b) | c) | d) |
|---|---|---|---|---|
| Wirkstoff | 80% | 10% | 5% | 95% |
| Aethylenglykol-monomethyl-äther | 20% | - | - | - |
| Polyäthylenglykol M G 400 | - | 70% | - | - |
| N-Methyl-2-pyrrolidon | - | 20% | - | - |
| Epoxydiertes Kokosnussöl | - | - | 1% | 5% |
| Benzin (Siedegrenzen 160-190°C) | - | - | 94% | - |

Die Lösungen sind zur Anwendung in Form kleinster Tropfen geeignet.

| 3. Granulate | a) | b) |
|---|---|---|
| Wirkstoff | 5% | 10% |
| Kaolin | 94% | - |
| Hochdisperse Kieselsäure | 1% | |
| Attapulgit | - | 90% |

Der Wirkstoff wird in Methylenchlorid gelöst, auf den Träger aufgesprüht und das Lösungsmittel anschliessend im Vakuum abgedampft.

| 4. Stäubemittel | a) | b) |
|---|---|---|
| Wirkstoff | 2% | 5% |
| Hochdisperse Kieselsäure | 1% | 5% |
| Talkum | 97% | - |
| Kaolin | - | 90% |

Durch inniges Vermischen der Trägerstoffe mit dem Wirkstoff erhält man gebrauchsfertige Stäubemittel.

Formulierungsbeispiele für feste Wirkstoffe der Formel I
(% = Gewichtsprozent)

| 5. Spritzpulver | a) | b) |
|---|---|---|
| Wirkstoff | 20% | 60% |
| Na-Ligninsulfonat | 5% | 5% |
| Na-Laurylsulfat | 3% | - |
| Na-Diisobutylnaphthalinsulfonat | - | 6% |
| Octylphenolpolyäthylenglykoläther (7-8 Mol AeO) | - | 2% |
| Hochdisperse Kieselsäure | 5% | 27% |
| Kaolin | 67% | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| 6. Emulsions-Konzentrat | |
|---|---|
| Wirkstoff | 10% |
| Octylphenolpolyäthylenglykoläther (4-5 Mol AeO) | 3% |
| Ca-Dodecylbenzolsulfonat | 3% |
| Ricinusölpolyglykoläther (36 Mol AeO) | 4% |
| Cyclohexanon | 30% |
| Xylolgemisch | 50% |

Aus diesem Konzentrat können durch Verdünnen mit Wasser Emulsionen jeder gewünschten Konzentration hergestellt werden.

| 7. Stäubemittel | a) | b) |
|---|---|---|
| Wirkstoff | 5% | 8% |
| Talkum | 95% | - |
| Kaolin | - | 92% |

Man erhält anwendungsfertige Stäubemittel, indem der Wirkstoff mit den Träger vermischt und auf einer geeigneten Mühle vermahlen wird.

| 8. Extruder Granulat | |
|---|---|
| Wirkstoff | 10% |
| Na-Ligninsulfonat | 2% |
| Carboxymethylcellulose | 1% |
| Kaolin | 87% |

Der Wirkstoff wird mit den Zusatzstoffen vermischt, vermahlen und mit Wasser angefeuchtet. Dieses Gemisch wird extrudiert und anschliessend im Luftstrom getrocknet.

| 9. Umhüllungs-Granulat | |
|---|---|
| Wirkstoff | 3% |
| Polyäthylenglykol (M G 200) | 3% |
| Kaolin | 94% |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

| 10. Suspensions-Konzentrat | |
|---|---|
| Wirkstoff | 40% |
| Aethylenglykol | 10% |
| Nonylphenolpolyäthylenglykoläther ( 15 Mol AeO) | 6% |

| | |
|---|---|
| Na-Lingninsulfonat | 10% |
| Carboxymethylcellulose | 1% |
| 37%ige wässrige Formaldehyd-Lösung | 0,2% |
| Silikonöl in Form einer 75%igen wässrigen Emulsion | 0,8% |
| Wasser | 32% |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

**Beispiel 1:** **Herstellung der Verbindung der Formel**

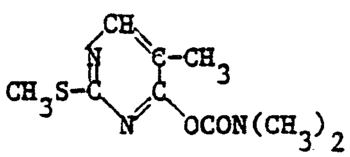

a) Herstellung von 2-Thiol-4-hydroxy-5-methyl-pyrimidin.

284 g frisch destillierter α-Formylpropionsäuremethylester, 186,3 g Thioharnstoff und 338 g $K_2CO_3$ werden in 4500 ml Aethanol 12 Stunden lang am Rückfluss gekocht. Der dicke Brei wird heiss abgenutscht; das Nutschgut bei 60°C in 200 ml Wasser gelöst und mit konz. $H_2SO_4$ auf pH 2 gestellt. Nach dem Abfiltrieren bei 50°C und der Reinigung durch Lösen in warmer Natriumkarbonatlösung und dem Fällen mit $H_2SO_4$ erhält man die Verbindung der Formel

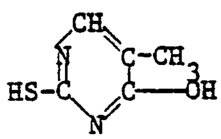

mit einem Schmelzpunkt von 280-290°C.

b) Herstellung von 2-Methylthio-4-hydroxy-5-methyl-pyrimidin.

142,2 g 2-Thiol-4-hydroxy-5-methyl-pyrimidin werden in 845 ml Methanol und 630 ml Wasser vorgelegt und auf 0°C abgekühlt. In diese

Lösung werden bei 0°C 42 g $LiOH \cdot H_2O$ zugegeben und dann 74,7 ml Methyljodid zugetropft. Nach 5 Stunden Ausrühren bei 20°C wird das ausgefallene Produkt abgetrennt. Man erhält, nach dem gründlichen Waschen des Produktes mit Wasser und Trocknen, die Verbindung der Formel

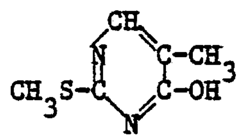

mit einem Schmelzpunkt von 225-227°C.

Auf analoge Weise werden auch folgende Verbindungen hergestellt:

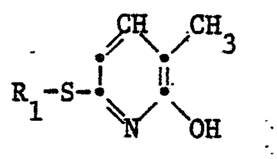

| $R_1$ | Smp. |
|---|---|
| $CH_3CH_2-S-$ | 140-147°C |
| 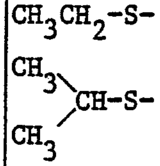 | 134-140°C |
| $Cl_3C-CH_2-CH_2-$ | 135-138°C |
| 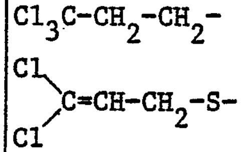 | 117-127°C |
| $HC\equiv C-CH_2-S-$ | 167-171°C |
| $CNCH_2CH_2-S-$ | 178-180°C |

c) Herstellung von 2-Methylthio-4-N,N-dimethyl-carbamoyloxy-5-methyl-pyrimidin.

15,6 g 2-Methylthio-4-hydroxy-5-methyl-pyrimidin, 9,2 ml Dimethylcarbamoylchlorid, 13,8 ml Triäthylamin und 100 mg p-Dimethylaminopyridin werden 14 Stunden lang in Chloroform am Rückfluss gehalten. Nach dem Abfiltrieren des Unlöslichen und dem Entfernen des Lösungsmittels wird das Rohprodukt in Aether/Pentan umkristallisiert.

Man erhält die Verbindung der Formel

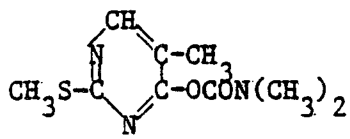

mit einem Schmelzpunkt von 82-85°C.

Auf analoge Weise werden auch folgende Verbindungen hergestellt:

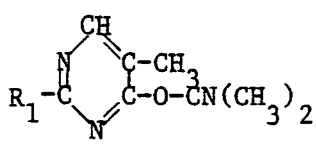

| $R_1$ | |
|---|---|
| 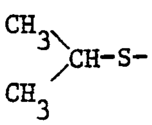 | $n_D^{20°}$ = 1,5425 |
| $C_2H_5$-S- | $n_D^{20°}$ = 1,5483 |
| $Cl_3C-CH_2-CH_2$-S- | Smp.: 85-95°C |
| $CH{\equiv}C-CH_2$-S- | Smp.: 105-110°C |
| 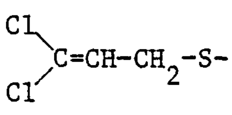 | Smp.: 30-34°C |

d) Herstellung von 2-Methylsulfonyl-4-N,N-dimethyl-carbamoyloxy-5-methyl pyrimidin.

15 g 2-Methylthio-4-N,N-dimethyl-carbamoyloxy-5-methyl-pyrimidin werden in 50 ml Chloroform vorgelegt und 25 g m-Chlorperbenzoesäure gelöst in 175 ml Chloroform bei 0-5°C langsam zugetropft. Nach 12 stündigem Rühren bei Raumtemperatur wird filtriert, die Lösung mit Soda gewaschen, über $Na_2SO_4$ getrocknet und eingeengt. Das Rohprodukt wird an wenig Kieselgel mittels Methylenchlorid gereinigt.

Man erhält die Verbindung der Formel

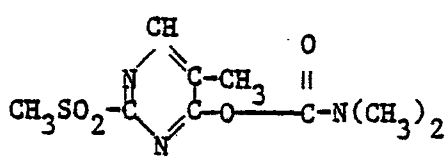

mit einer Refraktion von $n_D^{20°} = 1,5268$

e) Herstellung von 2-Methylsulfinyl-4-N,N-dimethyl-carbamoyloxy-5-methyl-pyrimidin. In 15 g 2-Methylthio-4-N,N-dimethylcarbamyloxy-5-methyl-pyridin in 75 ml Chloroform werden bei -15°C 11,4 g m-Chlorperbenzoesäure in 100 ml Chloroform langsam zugetropft. Nach 12 stündigem Rühren bei Raumtemperatur wird filtriert, die Lösung mit Soda gewaschen, über $Na_2SO_4$ getrocknet und eingeengt. Das Rohprodukt wird an wenig Kieselgel mittels Methylenchlorid gereinigt. Man erhält die Verbindung der Formel

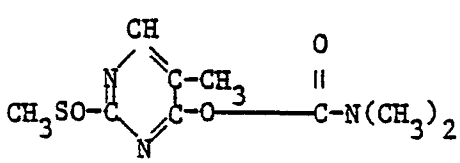

mit einer Refraktion von $n_D^{20°} = 1,5548$

Beispiel 2: Insektizide Kontakt-Wirkung: Aphis craccivora und Myzus persicae.

In Töpfen angezogene Pflanzen (Vicia fabae) wurden vor dem Versuchsbeginn je mit ca. 200 Individuen der Spezies Aphis fabae oder Myzus persicae besiedelt. Die so behandelten Pflanzen wurden 24 Stunden später mit einer Lösung enthaltend 200 oder 100 ppm der zu prüfenden Verbindung bis zur Tropfnässe besprüht. Man verwendete pro Test-Verbindung und pro Konzentration zwei Pflanzen und eine Auswertung der erzielten Abtötungsrate erfolgte nach weiteren 24 Stunden.

Die Verbindung gemäss Beispiel 1 zeigte im obigen Versuch eine positive Wirkung gegen Insekten der Spezies Aphis craccivora und Myzus persicae.

Beispiel 3: Systemisch insektizide Wirkung gegen Aphis craccivora.

In durch einen Plastikdeckel verschlossene Behälter wurden je 20 ml wässrige Lösung (Konzentration 25; 5 und 1 ppm Aktivsubstanz) gegeben. Durch ein Loch im Plastikdeckel wurden die Wurzeln von mit Läusen infizierten Erbsenkeimlingen in die Versuchslösung gebracht. Um den Einfluss der Gasphase der Lösung anszuschliessen wurde das Loch im Plastikdeckel mit Watte zusätzlich abgedichtet.
Nach 2 Tagen wurde auf saugfähige Tiere bonitiert.

Die Verbindungen gemäss Beispiel 1 zeigten im obigen Versuch eine gute systemische Wirkung gegen Insekten der Spezies Aphis craccivora. (Siehe Tabelle)

| Testverbindungen 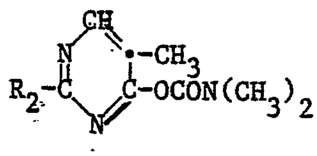 | Minimalkonzentration in ppm zur 100%igen Abtötung von Aphis craccivora |
|---|---|
| $R_2$ | |
| $CH_3-S-$ | 1 |
| 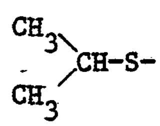 | 1 |
| $C_2H_5-S-$ | 1 |
| $Cl_3C-CH_2-CH_2-S-$ | 25 |
| $CH{\equiv}C-CH_2-S-$ | 5 |
| 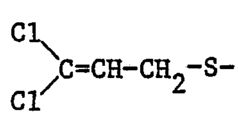 | 25 |
| $CH_3SO_2-$ | 1 |
| $CH_3SO-$ | 1 |

Patentansprüche

1. Eine Verbindung der Formel

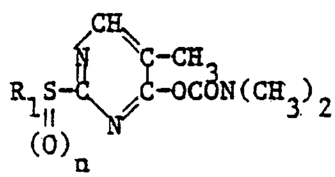

worin $R_1$ $C_1$-$C_4$ Alkyl, $C_1$-$C_4$-Alkylthio-$C_1$-$C_4$-alkyl, $C_1$-$C_4$-Alkoxy-$C_1$-$C_4$-alkyl, $C_1$-$C_4$-Halogenalkyl, $C_1$-$C_4$-Cyanoalkyl, $C_2$-$C_5$-Alkenyl, $C_2$-$C_5$-Halogenalkenyl oder $C_2$-$C_5$-Alkinyl und n die Zahlen 0, 1 oder 2 bedeuten.

2. Eine Verbindung gemäss Anspruch 1, worin $R_1$ $C_1$-$C_4$-Alkyl, $C_1$-$C_3$-Halogenalkyl, $C_1$-$C_3$-Cyanoalkyl, Methylthiomethyl, 3,3-Dimethyl-2-allyl, 3,3-Dichlor-2-allyl oder Propargyl und n die Zahlen 0, 1 oder 2 bedeuten.

3. Eine Verbindung gemäss Anspruch 1, worin $R_1$ Methyl, Aethyl, Propyl, Isopropyl, i-Butyl, 3,3,3-Trichlorpropyl, Cyanomethyl, Cyanoäthyl, Methylthiomethyl, 3,3-Dimethyl-2-allyl, 3,3-Dichlor-2-allyl oder Propargyl und n die Zahl 0 bedeuten.

4. Die Verbindung gemäss Anspruch 3 der Formel

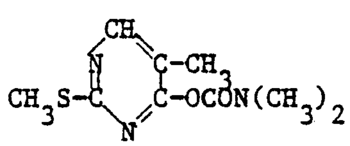

5. Die Verbindung gemäss Anspruch 2 der Formel

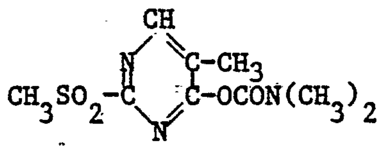

6. Die Verbindung gemäss Anspruch 2 der Formel

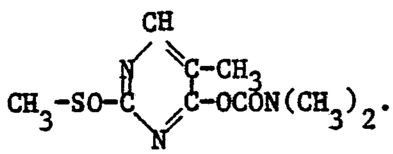

7. Ein Verfahren zur Herstellung von Verbindungen gemäss Anspruch 1, dadurch gekennzeichnet, dass man eine Verbindung der Formel

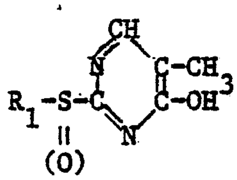

in Gegenwart von Basen mit Dimethylcarbamoylchlorid umsetzt, worin $R_1$ und n die im Anspruch 1 angegebene Bedeutung haben.

8. Ein Schädlingsbekämpfungsmittel, welches als aktive Komponente eine Verbindung gemäss Anspruch I und geeignete Träger- und/oder andere Zuschlagstoffe enthält.

9. Verwendung einer Verbindung gemäss Anspruch 1 zur Bekämpfung von Schädlingen an Tieren und Pflanzen.

10. Verwendung gemäss Anspruch 9 zur Bekämpfung von Insekten und Vertretern der Ordnung Akarina.

11. Eine Verbindung der Formel

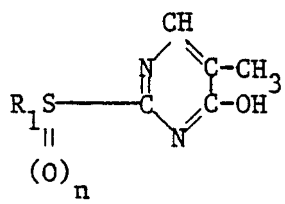

worin $R_1$ $C_1$-$C_4$ Alkyl, $C_1$-$C_4$-Alkylthio-$C_1$-$C_4$-alkyl, $C_1$-$C_4$-Alkoxy-$C_1$-$C_4$-alkyl, $C_1$-$C_4$-Halogenalkyl, $C_1$-$C_4$-Cyanoalkyl, $C_2$-$C_5$-Alkenyl, $C_2$-$C_5$-Halogenalkenyl oder $C_2$-$C_5$-Alkinyl und n die Zahlen 0, 1 oder 2 bedeuten.